(19) Europäisches Patentamt
European Patent Office
Office européen des brevets

(11) **EP 1 827 218 B1**

(12) **EUROPÄISCHE PATENTSCHRIFT**

(45) Veröffentlichungstag und Bekanntmachung des Hinweises auf die Patenterteilung:
**24.08.2011 Patentblatt 2011/34**

(21) Anmeldenummer: **05850193.3**

(22) Anmeldetag: **23.12.2005**

(51) Int Cl.:
***A61B 5/04*** *(2006.01)*

(86) Internationale Anmeldenummer:
**PCT/DE2005/002319**

(87) Internationale Veröffentlichungsnummer:
**WO 2006/066566 (29.06.2006 Gazette 2006/26)**

(54) **Verfahren zur Minimierung des Einflusses von Bewegungsartefakten eines Sensorsystems mit einer kapazitiven Elektrode**

Methods for reducing influences resulting from movement artefacts of a sensor system comprising a capacitive electrode

Procédés pour réduire les artefacts de mouvement d'un système de détection comprenant une électrode capacitive

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.12.2004 DE 102004063249**

(43) Veröffentlichungstag der Anmeldung:
**05.09.2007 Patentblatt 2007/36**

(73) Patentinhaber:
• **Fraunhofer-Gesellschaft zur Förderung der angewandten Forschung e.V.
80686 München (DE)**
• **Technische Universität Braunschweig
38106 Braunschweig (DE)**

(72) Erfinder:
• **MÜLLER, Klaus-Robert
12159 Berlin (DE)**
• **BLANKERTZ, Benjamin
10247 Berlin (DE)**
• **CURIO, Gabriel
14167 Berlin (DE)**
• **SCHILLING, Meinhard
38302 Wolfenbüttel (DE)**

(74) Vertreter: **Gross, Felix
Patentanwälte Maikowski & Ninnemann
Postfach 15 09 20
10671 Berlin (DE)**

(56) Entgegenhaltungen:
**EP-A- 1 428 471          WO-A-2004/052190
US-A1- 2004 073 104     US-A1- 2004 254 435
US-B1- 6 445 940          US-B1- 6 807 438**

• **HARLAND C J ET AL: "Remote detection of human electroencephalograms using ultrahigh input impedance electric potential sensors" APPLIED PHYSICS LETTERS AIP USA, Bd. 81, Nr. 17, 21. Oktober 2002 (2002-10-21), Seiten 3284-3286, XP012032293 ISSN: 0003-6951**
• **SEARLE A ET AL: "A direct comparison of wet, dry and insulating bioelectric recording electrodes; A comparison of bioelectrode performance" PHYSIOLOGICAL MEASUREMENT, INSTITUTE OF PHYSICS PUBLISHING, BRISTOL, GB, Bd. 21, Nr. 2, 1. Mai 2000 (2000-05-01), Seiten 271-283, XP020073475 ISSN: 0967-3334**

**Beschreibung**

Die Erfindung betrifft zwei Verfahren zur kapazitiven Messung elektromagnetischer Signale biologischen Ursprungs.

Ein solches Verfahren verwendet ein Sensorsystem zur kapazitiven Messung elektromagnetischer Signale biologischen Ursprungs, das eine kapazitiven Elektrodeneinrichtung, ein die Elektrodeneinrichtung zumindest teilweise umschließendes Elektroden-Abschirmelement zur Abschirmung der Elektrodeneinrichtung vor externen elektromagnetischen Störfeldern und eine Signalverarbeitungseinrichtung zur Verarbeitung elektromagnetischer Signale, die sich mittels der Elektrodeneinrichtung detektieren lassen, umfasst. Solche Sensorsysteme kommen üblicherweise in der Medizintechnik zum Einsatz, insbesondere um Signale biologischen Ursprungs für Elektroenzephalogramme (EEG) und Elektrokardiogramme (EKG) aufzunehmen.

Die kapazitive Messung der elektromagnetischen Signale biologischen Ursprungs weist gegenüber der aus dem Stand der Technik bekannten Methode des Einsatzes galvanisch mit einem Messobjekt gekoppelter Elektrodeneinrichtungen eine Reihe von Vorteilen auf. Insbesondere bei der Aufnahme eines EEGs entfällt die oftmals langwierige Vorarbeit, Messbereiche auf dem Kopf einer Testperson von Haaren frei zu machen und den elektrischen Widerstand der Kopfhaut in diesen Bereichen beispielsweise durch den Einsatz eines Peeling-Mittels zusätzlich zu den ohnehin benötigten Elektroden-Gels herabzusetzen. Bei einer kapazitiven Kopplung zwischen einem Messbereich der Testperson und einer Elektrodeneinrichtung spielt der elektrische Widerstand des Kopplungsbereichs keine Rolle mehr. Aus der US 2003/0036691 A1 und aus der WO 03/048789 A2 sind jeweils gattungsgemäße kapazitive Sensorsysteme bekannt.

Da die zu ermittelnden Messsignale biologischen Ursprungs sehr klein sind, reagieren die aus dem Stand der Technik bekannten kapazitiven Sensorsysteme zur Messung elektromagnetischer Signale biologischen Ursprungs trotz des vorhandenen Elektroden-Abschirmelements empfindlich auf externe elektromagnetische Störfelder. Darüber hinaus ergibt sich das Problem, dass sich die Elektrodenkapazität, die bedingt durch die Anordnung des Sensorsystems am Messobjekt zwischen der kapazitiven Elektrodeneinrichtung des Sensorsystems und dem Messobjekt gebildet ist, durch die Bewegung des Messobjekts relativ zur Elektrodeneinrichtung ändert, so dass das durch die Elektrodeneinrichtung erfasste elektromagnetische Signal durch Bewegungsartefakte überlagert und gestört ist.

Bei einem aus der US 2004/254435 A1 bekannten System zur Messung elektromagnetischer Signale biologischen Ursprungs ist eine Elektrode von einem Gehäuse eingefasst und mittels eines zusätzlichen Abschirmmittels von einer Signalverarbeitungseinrichtung abgeschirmt.

Aus der US 2004/073104 A1 ist eine Bio-Elektrode bekannt, die eine Polsterschicht aus einem dielektrischen Material aufweist, die zwischen dem Körper, auf dem das biologische Signal zu messen ist, und der Elektrodeneinrichtung angeordnet ist. Die Elektrodeneinrichtung, bestehend aus der Elektrode und der Signalverarbeitungseinrichtung, ist von einem abschirmenden Gehäuse umgeben, und die Signalverarbeitungseinrichtung ist durch ein Board von der Elektrode getrennt.

Aus der Veröffentlichung C. J. Harland et al., "Remote detection of human electroencephalograms using ultrahigh input impedance electric potential sensors", Applied Physics Letters, Vol. 81, Nr. 17, 21.10.2402 ist bekannt, kapazitive Elektrodeneinrichtungen zur Messung elektrischer Aktivität im menschlichen Gehirn zu verwenden, wobei Elektrodeneinrichtungen in einem speziell dem Kopf eines Menschen angepassten Helm angeordnet sind.

Die US 6,445,940 B1 bezieht sich auf eine kapazitive Elektrodenvorrichtung für die Messung von Gehirnwellen in einem EEG-System. Die US 6,445,940 B1 offenbart hierbei ein Verfahren zur Ermittlung der Kontaktqualität der Elektrodenanordnung an einem Patienten. Hierzu wird eine Stufenfunktion von wenigen Millivolt an die Elektrode angelegt und das Abklingverhalten der Stufenfunktion gemessen. Aus dem Abklingverhalten kann dann auf die Kapazität zwischen der Elektrode und dem Patienten rückgeschlossen werden und somit die Anordnung der Elektrode am Patienten überprüft werden.

Der vorliegenden Erfindung liegt somit die Aufgabe zu Grunde, ein verbessertes Sensorsystem zu schaffen.

Diese Aufgabe wird durch ein Verfahren mit den Merkmalen des Anspruchs 1 gelöst.

Bei einer kapazitiven Messung elektromagnetischer Signale biologischen Ursprungs tritt das Problem auf, dass selbst sehr geringe Relativbewegungen zwischen dem kapazitiven Sensorsystem und einer Signalquelle zu deutlichen Störsignalen führen. So reicht bereits die durch die Herzbewegung eines Organismus hervorgerufene periodisch auftretende mechanische Pulswelle dazu aus, das Messsignal zu beeinflussen. Darüber hinaus führen jedoch auch zwischen Sensorsystem und Signalquelle angeordnete Kleidung, Haare etc. bei einer Bewegung der Signalquelle zwangsläufig zu so genannten Bewegungsartefakten.

Um die aus dem Stand der Technik bekannten kapazitiven Sensorsysteme zu optimieren, ist es unumgänglich, den Einfluss der Bewegungsartefakte auf die Messsignale zu minimieren.

Bei der Verwendung eines Sensorsystems bzw. einer Messvorrichtung ist vorgesehen, das Sensorsystem oder die Messvorrichtung an einem Messobjekt anzuordnen. Danach wird ein elektrisches Wechselsignal in das Messobjekt eingekoppelt, um mittels der über die Elektrodeneinrichtung detektierten zeitlichen Änderung des Wechselsignals die Elektrodenkapazität der Elektrodeneinrichtung des Sensorsystems oder die Elektrodenkapazität der Elektrodeneinrichtungen der Messvorrichtung zu ermitteln. Diese ermittelte Elektrodenkapazität wird in einem abschließenden Schritt bei der Auswertung der Messsignale des Sensorsystems oder der Messvorrichtung berücksichtigt.

Es ist ebenso denkbar, dass sich das eingekoppelte Wechselsignal auch über eine andere Vorrichtung als die verwendeten Sensorsysteme auskoppeln und zur Elektradenkapazitätsermittlung auswerten lässt.

Die Einkopplung erfolgt beispielsweise über eine dafür am Messobjekt vorgesehene separate Elektrode. Diese Elektrode kann sowohl als ohmsche als auch als kapazitive Elektrode ausgebildet sein. Die Frequenz des eingekoppelten Wechselsignals liegt üblicherweise über eine Größenordnung entfernt von den Frequenzen der physiologisch relevanten Messsignale. Die Auskopplung des Wechselsignals kann auf technisch bekannte Weise mittels einer Lock-In-Verstärkerschaltung vorgenommen werden.

Bevorzugt wird das elektrische Wechselsignal zur Ermittlung der Elektrodenkapazität der Elektrodeneinrichtung des Sensorsystems oder zur Ermittlung der Elektrodenkapazität der Elektrodeneinrichtungen der Messvorrichtung über die Elektrodeneinrichtung oder über eine externe mit dem Sensorsystem oder der Messvorrichtung zusammenwirkende Referenzelektrode in das Messobjekt eingekoppelt. Die Referenzelektrode erfüllt in diesem Fall eine Doppelfunktion.

Der Einfluss der Bewegungsartefakte auf ein Sensorsystem zur Messung elektromagnetischer Signale biologischen Ursprungs lässt sich somit ganz allgemein durch ein Verfahren mit den folgenden Schritten minimieren. Zunächst wird ein zur Messung elektromagnetischer Signale biologischen Ursprungs geeignetes kapazitives Sensorsystems an einem Messobjekt angeordnet. Dann wird ein elektrisches Wechselsignals in das Messobjekt eingekoppelt, dann das eingekoppelte Wechselsignal zur Ermittlung der Elektrodenkapazität des Sensorsystems ausgewertet, und abschließend wird bei der Auswertung der Messsignale die ermittelte Elektrodenkapazität berücksichtigt.

Bevorzugt lässt sich eines der vorangehend genannten Verfahren derart durchführen, dass als elektrisches Wechselsignal ein netzfrequentes Störsignal benutzt wird. Das 50 bzw. 60Hz-Signal der Stromversorgung ist ohnehin vorhanden und müsste nicht erst mittels einer eigens dazu vorgesehenen Einrichtung generiert werden.

Mittels des Verfahrens wird die relative zeitliche Änderung der Elektrodenkapazität der Elektrodeneinrichtung des Sensorsystems oder der Messvorrichtung berücksichtigt und aus der relativen zeitlichen Änderung der Elektrodenkapazität die Bewegung der Elektrodeneinrichtung relativ zum Messobjekt abgeleitet. Mittels der so bestimmten Bewegung lassen sich dann die die elektromagnetischen Signale biologischen Ursprungs überlagernden Bewegungsartefakte bestimmen und kompensieren.

Ein weiteres Verfahren unter Verwendung eines Sensorsystems bzw. einer Messvorrichtung sieht die Schritte gemäß Anspruch 7 vor Zunächst wird das Sensorsystem oder die Messvorrichtung an einem Messobjekt angeordnet. Anschließend werden die Positionsparameter der Lage des Sensorsystems oder der Sensorsysteme relativ zum Messobjekt während der Messung ermittelt und die ermittelten Positionsparameter zur Kompensation von Bewegungsartefakten im Messsignal berücksichtigt.

Zur Ermittlung der benötigten Positionsparameter sind alle Sensorsysteme mit Positionsmesssystemen versehen. Diese Positionsmesssysteme ermitteln die benötigte Relativposition über ein geeignetes Messverfahren. Dazu eignen sich insbesondere optische, akustische und piezoelektrische Vorrichtungen und Verfahren unter Verwendung dieser Vorrichtungen.

Für die Verarbeitung der elektromagnetischen Signale, die sich mit der Elektrodeneinrichtung messen lassen, werden bevorzugt robuste Methoden der digitalen Signalverarbeitung eingesetzt. Insbesondere werden die Daten gefiltert, sowohl räumlich als auch im Frequenzbereich. Dabei können alle Filter auch während der Messung, gegebenenfalls in Echtzeit, an die momentane Signalcharakteristik adaptiert werden. Des Weiteren werden insbesondere univariate Entrauschungsverfahren eingesetzt, die auf der Zerlegung der Signale in beliebige - auch über- oder unterbestimmte - Basissysteme, wie z.B. Wavelets, Sinusfunktionen etc. beruhen. Univariate Entrauschung bedeutet, dass ein Messsignal des Sensorsystems für sich genommen isoliert von parallelen anderen Messsignalen des Sensorsystems entrauscht wird.

Insbesondere können auf Techniken zur Beschreibung der Signaldynamik (z.B. Auto-Regressiv-Koeffizienten, nichtlineare Dynamikparameter-Extraktionsverfahren) oder der Synchronizität eingesetzt werden, um geeignete Signalmerkmale zu extrahieren.

Des weiteren werden auch multivariate Methoden zur Entrauschung verwendet. Dabei werden mehrere Messsignale des Sensorsystems in einem gemeinsamen Prozess entrauscht. Diese Prozesse beruhen auf einer räumlichen Projektion der Messdaten, z.B. mit Hauptkomponentenanalyse, Independent-Component-Analysis, Projection-Pursuit-Techniken, Sparse-Decomposition-Techniken oder Bayesschen-Sub-Space-Regularization-Techniken.

Außerdem kommen Projektionstechniken zum Einsatz, die die Geometrie des Sensorsystems, insbesondere der Elektrodeneinrichtung und der Abschirmmittel bzw. des Elektroden-Abschirmelementes berücksichtigen, wie z.B. Beamforming-Techniken und Laplace-Filter.

Von den genannten räumlichen Projektionsmethoden können auch Varianten verwendet werden, die sich an Änderungen der Signalcharakteristika, so genannten Nichtstationaritäten, gegebenenfalls in Echtzeit, adaptieren. Unter Nichtstationaritäten werden ganz allgemein Änderungen der Umgebungsbedingungen verstanden. Zum Beispiel das Hinzukommen oder Wegfallen von Störquellen, Relativbewegungen zwischen Sensorsystem und Messobjekt, Veränderung des physiologischen Zustands des Messobjekts etc. Optional wird vor Beginn der eigentlichen Messung eine Kalibrationsmessung durchgeführt, bei der Signale unter bestimmten Bedingungen gemessen werden. Dies erlaubt den Einsatz überwachter Verarbeitungsverfahren, wie z.B. der Common-Spatial-Patterns-Technik zur räumlichen Projektion.

Die Kalibrationsdaten werden auch benutzt, um eine Modellselektion (Bestimmung des am besten geeigneten Verfahrens sowie der Werte der einstellbaren Parameter) durchzuführen.

Auf Basis dieser vorverarbeiteten, entrauschten Daten werden geeignete adaptive Techniken zur Klassifikation und Regression eingesetzt, die gegebenenfalls in Echtzeit an eine gegebenenfalls nichtstationäre Signalcharakteristik adaptieren. Beispiele solcher Verfahren sind lineare/nichtlineare Diskriminanz-Analyse, (Kern-)Fisher-Diskriminante, kernbasierte Lernverfahren (z.B. Support-Vektor-Maschinen, lineare Programmiermaschinen usw.), Boosting, Entscheidungsbäume und neuronale Netze.

Durch solche Techniken zur Klassifikation und Regression können beispielsweise aufgrund der gemessenen und vorverarbeiteten Messsignale unterschiedliche (Hirn-) Zustände unterschieden werden und somit Information übertragen werden. Es können auch Zustände vorhergesagt werden.

Ein Sensorsystem zur Verwendung im Rahmen des Verfahrens zur Minimierung des Einflusses von Bewegungsartefakten weist zusätzliche Abschirmmittel zur Abschirmung externer elektromagnetischer Störfelder auf, die die Elektrodeneinrichtung und das Elektroden-Abschirmelement räumlich zumindest teilweise umschließen. Die zusätzlichen Abschirmmittel sind dabei mit unterschiedlichen Kompartimenten ausgebildet, wobei in einem solchen Kompartiment die Signalverarbeitungseinrichtung angeordnet ist.

Dabei ist das Merkmal zusätzlicher Abschirmmittel im Rahmen der vorliegenden Erfindung so zu verstehen, dass neben dem Elektroden-Abschirmelement räumlich davon getrennt angeordnete zusätzliche Abschirmmittel vorgesehen sind. Räumlich getrennt ist jedoch nicht derart auszulegen, dass die zusätzlichen Abschirmmittel ohne mechanischen und/oder elektrischen Kontakt zu dem Elektroden-Abschirmelement angeordnet sind. Es kann also durchaus ein elektrischer Kontakt zwischen Abschirmmitteln und dem Elektroden-Abschirmelement, beispielsweise zur Gewährleistung eines identischen Potentials, vorgesehen sein.

Bevorzugt umschließen die zusätzlichen Abschirmmittel neben dem Elektroden-Abschirmelement die Signalverarbeitungseinrichtung zumindest teilweise. Dazu können die zusätzlichen Abschirmmittel sowohl derart ausgebildet sein, dass die Signalverarbeitungseinrichtung im Bereich zwischen den zusätzlichen Abschirmmittel und dem Elektraden-Abschirmelement angeordnet ist, als auch derart, dass die zusätzlichen Abschirmmittel sowohl das Elektroden-Abschirmelement als auch die Signalverarbeitungseinrichtung zumindest teilweise umschließen.

Die Signalverarbeitungseinrichtung ist somit durch die Abschirmmittel derart von der Signalquelle und von der Elecktrodeneinrichtung abgeschirmt, dass keine parasitären galvanischen, kapazitiven oder induktiven Einflüsse auf die Quelle oder auf die Elektrodeneinrichtung zurückwirken.

Auf diese Weise wird ein kompaktes Sensorsystem bereitgestellt, das im Hinblick auf die Abschirmung gegenüber externen elektromagnetischen Feldern optimiert ist. Besonders kompakt lässt sich das Sensorsystem in der Gestalt ausbilden, dass die Signalverarbeitungseinrichtung angrenzend, d.h. benachbart zum Elektroden-Abschirmelement angeordnet ist. Die räumliche Nähe zwischen Elektrodeneinrichtung und Signalverarbeitungseinrichtung bringt eine Reihe von Vorteilen mit sich, die im Folgenden noch näher erläutert wird.

Als Signalverarbeitungseinrichtung wird im Sinne der vorliegenden Erfindung jedes System betrachtet, das die eingehenden Messsignale verändert, d.h. das System tritt in eine Wechselwirkung mit den Messsignalen derart, dass die Messsignale nach dem Durchlaufen des Systems verändert sind.

Eine bevorzugte Variante des Sensorsystems sieht vor, dass die zusätzlichen Abschirmmittel die Signalverarbeitungseinrichtung zumindest teilweise derart umschließen, dass die zusätzlichen Abschirmmittel die Elektrodeneinrichtung gegenüber aus der Signalverarbeitungseinrichtung herrührender elektromagnetischer Störfelder abschirmt.

Außerdem ist es vorteilhaft, die zusätzlichen Abschirmmittel derart auszubilden, dass die Elektrodeneinrichtung und die Signalverarbeitungseinrichtung so überdeckt sind, dass ein Raumwinkelbereich definiert ist, aus dem herrührende elektromagnetische Signale ohne wesentliche Beeinflussung durch die Abschirmmittel und/oder das Elektroden-Abschirmelement mittels des Sensorsystems detektierbar sind.

Eine weitere Variante des Sensorsystems sieht vor, dass der Abstand zwischen dem Elektroden-Abschirmelement und der Elektrodeneinrichtung und/oder die Geometrie des Elektroden-Abschirmelements und der Elektrodeneinrichtung und/oder die dielektrischen Eigenschaften eines zwischen dem Elektroden-Abschirmelement und der Elektrodeneinrichtung angeordneten Füllmaterials derart gewählt sind, dass die daraus resultierende Abschirm-Kapazität zwischen der Elektrodeneinrichtung und dem Elektroden-Abschirmelement klein ist, um so die Einkopplung von Rauschsignalen der Signalverarbeitungseinrichtung in die Elektrodeneinrichtung zu minimieren. Auf diese Weise wird sichergestellt, dass der Signalpfad ausgehend von der Elektrodeneinrichtung in Richtung der Signalverarbeitungseinrichtung im Wesentlichen als "Einbahnstraße" funktioniert. Die Elektrodeneinrichtung, die mindestens ein Elektrodenelement umfasst, ist auf diese Weise gegenüber der Einkopplung von Störsignalen weitestgehend entkoppelt.

Im Hinblick auf die Geometrie der Elektrodeneinrichtung ist es weiterhin von Vorteil, diese derart zu wählen, dass die Elektrodenkapazität der Elektrodeneinrichtung relativ zu einem Messobjekt zusammen mit einer parasitären Eingangsimpedanz der Signalverarbeitungseinrichtung einen Hochpass mit einer an ein zu messendes elektromagnetisches Signal biologischen Ursprungs angepassten Grenzfrequenz ausbildet. Auch diese Maßnahme trägt zu der vorangehend genannten Entkopplung der Elektrodeneinrichtung gegenüber der Signalverarbeitungseinrichtung bei.

Im Hinblick auf die Geometrie der Elektrodeneinrichtung ist diese bevorzugt derart gewählt, dass Rauschsignale der parasitären Eingangsimpedanz der Signalveratbeitungseinrichtung mit ihrer oberen Grenzfrequenz unterhalb der unteren Grenzfrequenz für die zu messenden Signale liegen.

In einer vorteilhaften Variante umfasst das Sensorsystem ein Gehäuse. Dieses Gehäuse umfasst dabei die zusätzlichen Abschirmmittel. Das heißt, die Abschirmmittel sind als Bestandteil bzw. Bestandteile des Gehäuses ausgebildet. Die Bestandteile können dazu im Verhältnis zum Gehäuse sowohl einstückig als auch modular ausgebildet sein. Bevorzugt ist die Elektrodeneinrichtung derart mit dem Gehäuse gekoppelt, dass der ohmsche Widerstand zwischen der Elektrodeneinrichtung und dem Gehäuse derart hoch ist, dass ein von der Elektrodeneinrichtung kapazitiv aufgenommenes Signal am Eingang der Signalverarbeitungseinrichtung verfälschungsfrei anliegt.

Mit Vorteil ist die kapazitive Elektrodeneinrichtung derart im Gehäuse angeordnet, dass keine Verbindungen zu elektrischen Quellen auftreten können, die die Gefahr der Zerstörung der Elektrodeneinrichtung oder der Signalverarbeitungseinrichtung mit sich bringen. Dazu umfasst das Sensorsystem insbesondere einen elektrischen Isolatorbereich zur elektrischen Isolierung der Elektrodeneinrichtung gegenüber einer Signalquelle. Dieser ist mit Vorteil derart ausgebildet, dass sowohl das Elektroden-Abschirmelement als auch die Abschirmmittel bei einer Messung an einer Signalquelle von dieser Signalquelle galvanisch getrennt sind. Außerdem weist der Isolatorbereich bevorzugt derartige Materialeigenschaften auf, dass eine statische Aufladung des Isolatorbereichs durch statische Umgebungsladungen minimiert ist.

Die Signalverarbeitungseinrichtung des Sensorsystems umfasst als Eingangsstufe bevorzugt einen Impedanzwandler. In einer vorteilhaften Variante weist die Signalverarbeitungseinrichtung einen Differenzverstärker auf. Als Differenzsignal eignet sich dabei insbesondere das Signal einer externen Referenzelektrode, die mit der Signalquelle in Kontakt steht.

Eine besonders bevorzugte Variante des Sensorsystems zeichnet sich durch eine integrierte Analog-Digital-Wandlung der Signale aus. Dazu umfasst die Signalverarbeitungseinrichtung einen Analog-Digital-Wandler und einen digitalen Signalprozessor. Derartige Prozessoren sind in einem hinreichenden Miniaturisierungsgrad erhältlich, so dass auch in dieser Funktionalität ein entsprechend kompaktes Sensorsystem bereitgestellt werden kann.

Die Eingangsimpedanz der Signalverarbeitungseinrichtung ist mit Vorteil derart zu wählen, dass sich zusammen mit der Elektrodenkapazität des Elektrodensystems ein Hochpass mit einer an ein zu messendes elektromagnetisches Signal biologischen Ursprungs angepassten Grenzfrequenz ausbildet. Die Elektrodenkapazität des Elektrodensystems lässt sich insbesondere über die vorangehend erläuterten Geometrie-Parameter des Sensorsystems einstellen.

Bevorzugt ist in der Signalverarbeitungseinrichtung ein Hochpass derart vorgesehen, dass ein in der Signalverarbeitungseinrichtung verstärktes Signal den Hochpass durchläuft, so dass sich Gleichspannungspotentiale vom dynamischen Messsignal abtrennen.

Eine weitere Variante des Sensorsystems sieht vor, dass die Elektrodeneinrichtung eine Mehrzahl von Elektrodenelementen zum Detektieren elektromagnetischer Signale biologischen Ursprungs aufweist. Die Mehrzahl der Elektrodenelemente wirkt dabei als eine Mehrzahl kapazitiver Elektroden. Für jedes dieser Elektrodenelemente ist ein entsprechender Signalverarbeitungspfad in der Signalverarbeitungseinrichtung ausgebildet. Ebenso ist denkbar eine entsprechende Mehrzahl von Teil-Signalverarbeitungseinrichtungen vorzusehen.

Bevorzugt weist das Sensorsystem eine auf Massepotential abgeschirmte elektrische Leitungseinrichtung zum Wegführen des Messsignals vom Sensorsystem auf. Weitere Varianten des Sensorsystems verfügen über Mittel zum leitungsgebundenen und/oder Mittel zum leitungslosen Übertragen der Messsignale vom Sensorsystem zu einer davon beabstandeten Empfangseinrichtung. Die Signalübertragung lässt sich sowohl für die leitungsgebunderie als auch für die leitungslose Variante optisch und/oder elektrisch realisieren. Für die optische Variante verfügt das Sensorsystem über eine bevorzugt miniaturisiert ausgebildete optoelektronische Wandlungseinrichtung. Die Datenübertragung kann dann sowohl im Freistrahl als auch über Lichtwellenleiter, wie Lichtleitfasern aus Gläsern oder Kunststoffen erfolgen.

Es ist von Vorteil, das Sensorsystem mit einer Referenzelektrode auszubilden. Diese Referenzelektrode lässt sich neben dem Sensorsystem an einem Messobjekt derart anordnen, dass die Referenzelektrode ein Referenzpotential für das Sensorsystem bereitstellt. Die Referenzelektrode ist dabei bevorzugt als ohmsche Elektrode ausgebildet.

Prinzipiell ist es aber möglich, dass die Referenzelektrode resistiv, induktiv oder kapazitiv mit dem Messobjekt gekoppelt ist.

Neben der Bereitstellung eines Referenzpotentials kann die Referenzelektrode auch zur Einkopplung eines Wechselsignals verwendet werden, mittels dessen ein Bewegungssignal ableitbar Ist und damit Bewegungsartefakte im elektromagnetischen Signal biologischen Ursprungs kompensierbar sind. In diesem Fall erfüllt die Referenzelektrode eine Doppelfunktion, zum einen zur Bereitstellung des Referenzpotentials und zum anderen als Quelle für ein Wechselsignal, aus dem das Bewegungssignal ableitbar und dadurch Bewegungsartefakte kompensierbar sind.

Es können eine oder mehrere Referenzelektroden zur Einspeisung eines Wechselsignals verwendet werden.

Bei Verwendung mehrerer Referenzelektroden ist es dann möglich, Wechseisignale unterschiedlicher Frequenz einzukoppeln, wobei mittels jedes einzelnen Wechselsignals die Bewegungsartefakte unterschiedlicher Elektrodenelemente oder Elektrodeneinrichtungen getrennt kompensierbar sind.

Das vorangehend beschriebene Sensorsystem lässt sich in einer Vielzahl von Messvorrichtungen integrieren. Beispielhaft sollen im Folgenden zwei dargestellt werden, die sich insbesondere für die Aufnahme von EEGs bzw. EKGs eignen.

Die eine Messvorrichtung umfasst eine Vielzahl von Sensorsystemen, die in einer helm- oder kappenartigen Trägereinrichtung angeordnet sind. Diese Trägereinrichtung ist derart ausgebildet, das sie sich zumindest abschnittsweise über den Kopf einer Testperson stülpen lässt. Dabei weist sie für die Testperson bevorzugt Trageeigenschaften auf, die die Messungen über längere Zeiträume nicht unangenehm werden lassen. Das heißt das Gewicht, die Aufnahme oder die Durchlasseigenschaften für Körperfeuchtigkeit etc. sollten mit entsprechenden Materialien auf den Tragkomfort hin optimiert werden. Es ist denkbar, dass eine derartige Messvorrichtung in Form einer vorangehend beschriebenen Kappe insbesondere für die EEG-Diagnostik in der Notfallmedizin von großem Nutzen ist. Ebenso ist es denkbar, dass eine Person, die eine solche Kappe trägt, über ihre Himtätigkeit mit zu steuernden Systemen interagiert. Diese zu steuernden Systeme können Computer, künstliche Gliedmaßen, Roboter oder weitere durch einen Menschen zu überwachende oder zu steuernde Maschinen oder komplexe Systeme sein. Dabei würde die Kappe als Schnittstelle zwischen Mensch und Maschine dienen.

Im Freizeitbereich ließen sich auf diese Weise Computerspiele ganz oder teilweise steuern. Mittels eines zwischengeschalteten Computers wäre somit auch eine gedankliche Interaktion zwischen mehreren Menschen denkbar.

Eine zweite Messvorrichtung umfasst eine Vielzahl von Sensorsystemen, wobei die Sensorsysteme an einer flexiblen flächig ausgebildeten Trägereinrichtung angeordnet sind, die sich am Körper einer Testperson befestigen lässt. Damit eignet sich diese Messvorrichtung insbesondere zur Aufnahme von EKGs. Im Hinblick auf den Tragekomfort gelten die vorangehend gemachten Ausführungen entsprechend.

Damit eine an die individuelle Kopf- und Körperform anpassbare Flexibilität der vorangehend beschriebenen Messvorrichtungen gewährleistet ist, sind die Elektrodeneinrichtungen und/oder das Gehäuse und/oder die zusätzlichen Abschirmmittel und/oder die Elektroden-Abschirmelemente bevorzugt aus geeigneten flexiblen Kunststoffen hergestellt.

Weitere Eigenschaften und Vorteile der Erfindung werden im Zusammenhang mit den folgenden Zeichnungen erläutert. Es zeigen:

Figur 1a          eine schematische Querschnittsdarstellung einer ersten Ausfüh- rungsform des erfindungsgemäßen Sensorsystems;

Figur 1b          eine schematische Querschnittsdarstellung einer zweiten Ausfüh- rungsform des erfindungsgemäßen Sensorsystems;

Figur 2           ein schematisches Ersatzschaltbild des erfindungsgemäßen Sen- sorsystems;

Figuren 3a - 3c   drei Varianten zur mehrtelligen Ausgestaltung der Elektrodenein- richtung des Sensorsystems;

Figur 4           ein schematisches Ersatzschaltbild einer Ausführungsform einer Kompensationsschaltung zur Kompensation von statischen Ladun- gen auf der Elektrodeneinrichtung;

Figur 5           eine schematische Darstellung der Anordnung der Elektrodenein- richtung mit Abstand vom Messobjekt;

Figur 6           graphische Darstellung des Frequenzspektrums eines durch eine Bewegung der Elektrodeneinrichtung modulierten Wechselsignals;

Figur 7           graphische Darstellung eines durch eine Bewegung der Elektro- deneinrichtung modulierten Wechselsignals und des aus dem mo- dulierten Wechselsignal berechneten Bewegungssignals;

Figur 8           Ablaufschema eines Verfahrens zur Verfahren zur Minimierung des Einflusses von Bewegungsartefakten unter Verwendung eines Sen- sorsystems und

Figur 9           Ablaufschema eines zweiten Verfahrens zur Verfahren zur Minimie- rung des Einflusses von Bewegungsartefakten unter Verwendung eines Sensorsystems.

1. Aufbau des Sensorsystems

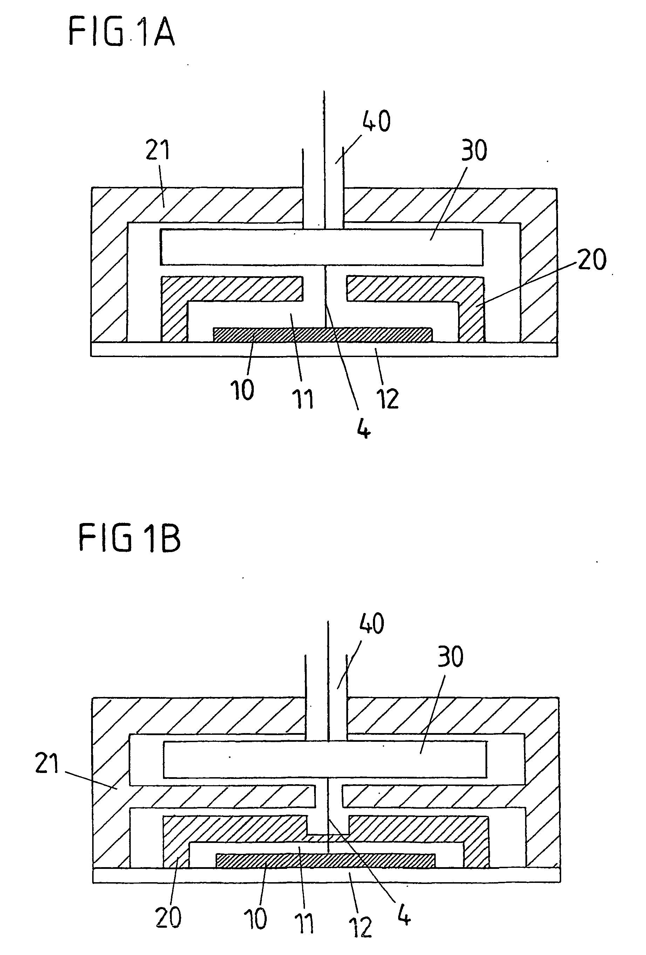

Figur 1a zeigt eine Querschnittsdarstellung einer ersten Ausführungsform des erfindungsgemäßen Sensorsystems. Diese Darstellung ist rein schematisch und nicht maßstabsgetreu. Auf einem als Isolatorbereich wirkenden flächig ausgebildeten Isolatorelement 12 ist die Elektrodeneinrichtung 10 angeordnet. Auf der der Elektrodeneinrichtung 10 zugewandten Seite des Isolatorelements 12 ist die Elektrodeneinrichtung 10 im Wesentlichen vollständig von einem Elektroden-Abschirmelement 20 umschlossen. Dieses Elektroden-Abschirmelement 20 ist ebenfalls auf dem Isolatorelement 12 angebracht und durch das Isolatorelement 12 von der Elektrodeneinrichtung 10 galvanisch entkoppelt.

Das Elektroden-Abschirmelement 20 weist eine Öffnung zur Durchführung einer von der Elektrodeneinrichtung 10 ausgehenden Signalleitung 4 auf. Diese Signalleitung 4 führt zu einer außerhalb des Elektroden-Abschirmelementes 20 angeordneten Signalverarbeitungseinrichtung 30. Sowohl die Signalverarbeitungseinrichtung 30 als auch das Elektroden-Abschirmelement 20 sind auf der der Elektrodeneinrichtung 10 zugewandten Seite des Isolatorelements 12 von zusätzlichen Abschirmmitteln 21 umschlossen. Die zusätzlichen Abschirmmittel 21 weisen eine Durchführung für eine auf Massepotential abgeschirmte elektrische Leitungseinrichtung 40 zum Wegführen des Messsignals vom Sensorsystem auf.

Die gezeigte Leitungseinrichtung 40 kann auch optisch in Form eines Lichtleiters ausgebildet sein. In einem solchen Falle umfasst die Signalverarbeitungseinrichtung 30 einen geeigneten elektro-optischen Wandler. Der Lichtleiter ließe sich dann sowohl als Lichtleitfaser als auch integriert optisch ausbilden. Die Verwendung eines Lichtleiters als Leitungseinrichtung 40 hätte den Vorteil, dass dieser keine Abschirmung gegenüber äußeren elektromagnetischen Feldern benötigen würde.

Das Isolatorelement 12 stellt zum einen eine galvanische Entkopplung der Elektrodeneinrichtung 10 sicher. Zum anderen dient es ebenso der galvanischen Entkopplung zwischen dem Elektroden-Abschirmelement 20 und den zusätzlichen Abschirmmitteln 21. In Figur 1a ist das Elektroden-Abschirmelement 20 im Querschnitt als zwei einander gegenüber liegend angeordnete L-förmige Schenkel ausgebildet. Es sind selbstverständlich eine Vielzahl anderer geometrischer Ausgestaltungen beispielsweise mit gewölbten Abschnitten des Elektroden-Abschirmelementes 20 möglich. Wesentlich ist jeweils, dass das Elektroden-Abschirmelement 20 die Elektrodeneinrichtung 10 derart umschließt, dass ein Raumwinkel definiert ist, aus dem kommend elektromagnetische Felder die Elektrodeneinrichtung 10 erreichen, ohne dabei eine durch das Elektroden-Abschirmelement 20 verursachte Dämpfung zu erfahren.

Die vorangehenden Ausführungen gelten sinngemäß im Hinblick auf die räumliche Ausgestaltung der zusätzlichen Abschirmmittel 21. Wie in Figur 1b dargestellt ist es möglich, die zusätzlichen Abschirmmittel 21 mit unterschiedlichen Kompartimenten auszubilden. In einem solchen Kompartiment lässt sich dann die Signalverarbeitungseinrichtung 30 derart anordnen, dass die zusätzlichen Abschirmmittel 21 die Elektrodeneinrichtung 10 gemeinsam mit dem Elektroden-Abschirmelement 20 auch gegenüber der Signalverarbeitungseinrichtung 30 abschirmen. Die Einstreuung in der Signalverarbeitungseinrichtung 30 generierter elektromagnetischer Felder wird auf diese Weise minimiert.

Es ist klar, dass eine Vielzahl geometrischer Ausgestaltungen sowohl der Elektroden-Abschirmelemente 20 als auch der zusätzlichen Abschirmmittel 21 existieren. Dies hängt insbesondere mit der räumlichen Ausgestaltung der Signalverarbeitungseinrichtung 30 zusammen. Die Signalverarbeitungseinrichtung 30 impliziert nicht, dass diese die vollumfängliche Verarbeitung der Messsignale vornehmen muss. Die Signalverarbeitung kann in der dargestellten Signalverarbeitungsvorrichtung 30 auch nur teilweise ablaufen. Der dargestellten Signalverarbeitungsvorrichtung 30 können weitere vom Sensorsystem entfernt angeordnete Signalverarbeitungsrichtungen nachgeordnet sein.

Ebenso gilt im Hinblick auf die zusätzlichen Abschirmmittel 21, dass diese nicht zwangsläufig einstückig ausgebildet sein müssen. Auch ein hybrider Aufbau aus einzelnen Abschirmelementen ist möglich. Ebenso können die Durchgangsöffnungen für die Signalleitungen 4 variabel aufgebaut sein, um unterschiedlichen Anforderungen an die Abschirmung zwischen Signalverarbeitungseinrichtung 30 und der Elektrodeneinrichtung 10 zu erfüllen.

Weiterhin kann die in den Figuren 1a und 1b als einheitliches Bauelement dargestellte Signalverarbeitungseinrichtung 30 aus einer Mehrzahl räumlich getrennter Teilelemente aufgebaut sein. Von diesen Teilelemente können einzelne oder alle durch die zusätzlichen Abschirmmittel 21 in unterschiedlichen oder gleichen Kompartimenten umschlossen sein.

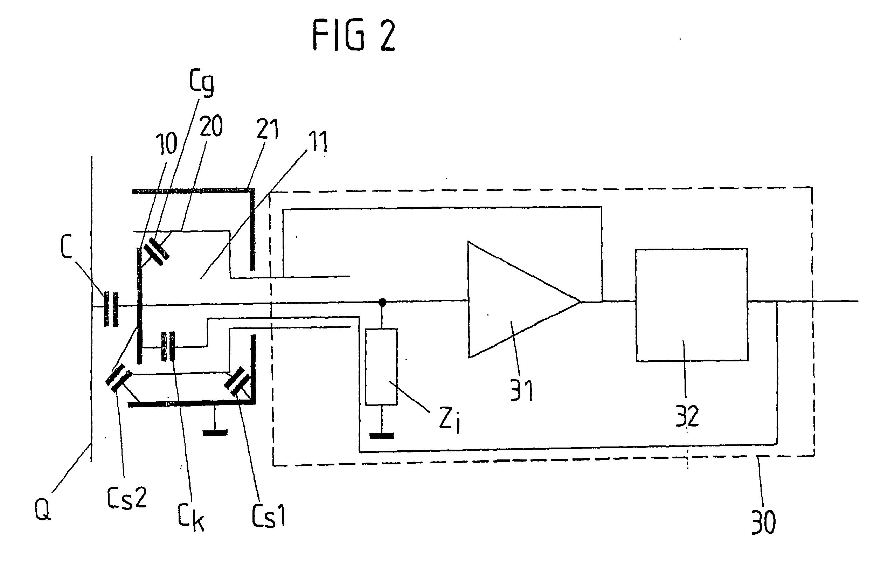

Figur 2 zeigt ein schematisches Ersatzschaltbild des erfindungsgemäßen Sensorsystems. Die Elektrodeneinrichtung 10 weist gegenüber einem Messobjekt Q, das als Quelle elektromagnetischer Signale biologischen Ursprungs wirkt, eine Elektrodenkapazität C auf.

Durch ein elektrisches Feld und das daraus resultierende elektrische Potential der Quelle Q wird auf der kapazitiven Elektrodeneinrichtung 10 gemäß dessen Kapazität C Ladung influenziert. Diese Ladung, die bei zeitabhängiger Quelle Q selbst auch zeitabhängig ist, gelangt auf einen als Impedanzwandler 31 wirkenden Operationsverstärker der Signalverarbeitungseinrichtung 30. Dieser Impedanzwandler 31 weist eine Eingangsimpedanz $Z_i$ auf. In dieser Eingangsimpedanz $Z_i$ sind alle resistiven, kapazitiven und induktiven externen Beiträge der Umgebung und die interne Eingangsimpedanz des Impedanzwandlers 31 zusammengefasst. Der externe Teil der Impedanz $Z_i$ soll einen möglichst kleinen kapazitiven und induktiven und einen möglichst großen resistiven Anteil aufweisen. Der Impedanzwandler 31 setzt sein Eingangssignal auf eine so kleine Ausgangsimpedanz um, dass konventionelle Schaltungen 32 zur weiteren Signalverarbeitung nachfolgend eingesetzt werden können. Das Ausgangssignal des Impedanzwandlers 31 stellt das Potential für das Elektroden-Abschirmelement 20 dar. Dieses Potential wird bei handelsüblichen Guard-Elektroden-Systemen als Guardpotential bezeichnet.

Bei der Aufnahme des Ladungssignals über die Kapazität C treten parasitäre Signale auf, die über eine zwischen Elektrodeneinrichtung 10 und Elektroden-Abschirmelement 20 wirkende parasitäre Abschirm-Kapazität Cg, über eine zwischen Elektroden-Abschirmelement 20 und den Abschirmmitteln 21 wirkende parasitäre erste Schirm-Kapazität Cs1 und über eine zwischen Elektrodeneinrichtung 10 und den AbSchirm-Kapazität Cs1 und über eine zwischen Elektrodeneinrichtung 10 und den Abschirmmitteln 21 wirkende parasitäre zweite Schirm-Kapazität Cs2 einkoppeln können.

Daher ist es vorteilhaft, die vorangehend beschriebenen parasitären Kapazitäten Cg, Cs1 und Cs2 durch eine entsprechende Anpassung geometrischer Parameter wie insbesondere die Abmaße und die Oberflächenkontur des Elektroden-Abschirmelements und der Abschirmmittel, der jeweilige Abstand zwischen Elektrodeneinrichtung 10, Elektroden-Abschirmelement 20 und Abschirmmitteln 21 einzustellen. Weiterhin lässt sich die parasitäre Kapazität Cg über die dielektrischen Eigenschaften des zwischen Elektrodeneinrichtung 10 und Elektroden-Abschirmelement 20 angeordneten Mediums 11 beeinflussen. Entsprechendes gilt selbstverständlich auch für die parasitären Kapazitäten Cs1 und Cs2.

Alternativ zur Rückkopplung des verarbeiteten Signals über die Kompensationsimpedanz Ck an die Elektrodeneinrichtung 10, lässt sich das verarbeitete Signal zusammen mit der Ausgangsleitung des Impedanzwandlers 31 verknüpfen, um aus einer geeigneten Verknüpfungsoperation das Potential für das Elektroden-Abschirmelement (Guardpotential) zu generieren. Welche Art der Signalverknüpfung (z.B. Differenzbildung, Addieren etc.) zur Erzeugung des Guardpotentials geeignet ist, hängt von den Parametern der Signalverarbeitung ab. Auch auf die vorangehend dargestellte Weise lässt sich der Dynamikbereich des Sensorsystems erhöhen.

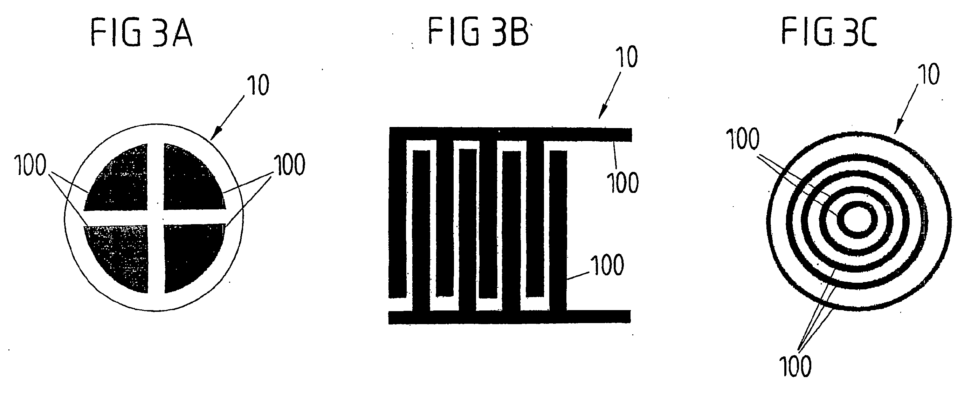

In den Figuren 3a bis 3c sind verschiedene Varianten zur Ausbildung der Elektrodeneinrichtung 10 dargestellt. Jede der drei gezeigten Varianten umfasst eine Mehrzahl von Elektrodenelementen 100.

In Figur 3a sind vier Elektrodenelemente in der Topologie symmetrisch angeordneter Viertelkreiselemente dargestellt. Figur 3b zeigt eine fingerartig ineinandergreifende Struktur zweier kammartiger Elektrodenelemente 100. In Figur 3c ist die Elektrodeneinrichtung 10 in Form von fünf als konzentrisch angeordnete Ringe unterschiedlichen Durchmessers angeordnete Elektrodenelemente 100 ausgeführt.

Bei den gezeigten mehrteiligen Ausführungsformen der Elektrodeneinrichtung 10 sind jeweils eine entsprechende Mehrzahl von Signalleitungen und Signalverarbeitungspfaden erforderlich, um eine parallele Signalverarbeitung in der Signalverarbeitungseinrichtung zu gewährleisten.

2. Kompensation von statischen Ladungen auf der Elektrodeneinrichtung

Äußere Ladungen in der Umgebung des Sensorsystems und der Elektrodeneinrichtung 10 des Sensorsystems bewirken, dass statische Ladungen auf der Elektrodeneinrichtung 10 oder den einzelnen Elektrodenelementen 100 der Elektrodeneinrichtung 10 erzeugt werden, sich dort sammeln und zu einer statischen Aufladung der Elektrodeneinrichtung 10 führen. Durch eine solche statische Aufladung der Elektrodeneinrichtung 10 oder der Elektrodenelemente 100 der Elektrodeneinrichtung 10 wird der dynamische Bereich des Sensorsystems zum Empfangen elektromagnetischer Signale aus dem Messobjekt Q erheblich beeinträchtigt und das erreichbare Signal-zu-Rausch-Verhältnis des Sensorsystems reduziert.

Die Erfassung elektromagnetischer Signale aus dem Messobjekt Q geht einher mit Ladungsverschiebungen auf der Elektrodeneinrichtung 10. Gelangt ein zu erfassendes elektromagnetisches Signal aus dem Messobjekt Q zur Elektrodeneinrichtung 10, so bewirkt das elektromagnetische Signal eine Ladungsverschiebung auf der Elektrodeneinrichtung 10, induziert einen Strom und somit ein Signal, das in der Signalverarbeitungseinrichtung 30 verarbeitet wird. Wenn jedoch auf der Elektrodeneinrichtung 10 statische Ladungen in Folge von äußeren Ladungen in der Umgebung des Sensorsystems präsent sind, so bewirkt dies, dass der dynamische Bereich der Elektrodeneinrichtung 10 für das eigentlich zu erfassende elektromagnetische Signal aus dem Messobjekt Q herabgesetzt ist und das elektromagnetische Signal zudem verstärkt von Störsignalen überlagert ist.

Zudem hat die auf der Elektrodeneinrichtung 10 befindliche statische Ladung einen erheblichen Einfluss auf die Störung des zu empfangenden elektromagnetischen Signals aus dem Messobjekt Q durch Bewegungsartefakte bedingt durch die Bewegung der Elektrodeneinrichtung 10 relativ zum Messobjekt Q. Die Änderung des durch die Elektrodeneinrichtung 10 empfangenen Signals in Abhängigkeit von der Entfernung der Elektrodeneinrichtung 10 zum Messobjekt Q lässt sich durch folgende Gleichung beschreiben:

$$\frac{\partial U}{\partial d} = \frac{1}{C}\frac{\partial Q}{\partial d} - \frac{Q}{C^2}\frac{\partial C}{\partial d} \qquad\qquad (1)$$

In der Gleichung (1) spiegelt der erste Term der Änderung der Spannung U der Elektrodeneinrichtung 10 mit dem Abstand d zwischen der Elektrodeneinrichtung 10 und dem

Messobjekt Q, der zweite Term der Änderung der Ladung Q mit dem Abstand d und der dritte Term der Änderung der Elektrodenkapazität C mit dem Abstand d wieder. Bei einem durch die Elektrodeneinrichtung 10 erfassten Signal U, das nicht durch Bewegungsartefakte gestört ist, müssen der zweite und dritte Term der Gleichung (1) Null sein, also keinen Beitrag liefern, so dass das Signal U unabhängig von der Änderung der Elektrodenkapazität C relativ zum Abstand d zwischen der Elektrodeneinrichtung 10 und dem Messobjekt Q ist. Wie aus Gleichung (1) ersichtlich ist, ist der dritte Term proportional zu der auf der Elektrodeneinrichtung 10 angesammelten Ladung Q. Eine Unterdrückung der auf der Elektrodeneinrichtung 10 angesammelten Ladung Q geht somit einher mit der Reduzierung von das empfangene Signal störenden Bewegungsartefakten.

Um die Ansammlung statischer Ladungen auf der Elektrodeneinrichtung 10 zu kompensieren, ist im Sensorsystem eine Rückkopplung zwischen dem Ausgang der Signalverarbeitungseinrichtung 30 und der Elektrodeneinrichtung 10 angeordnet. Bei dem in Figur 2 dargestellten Ersatzschaltbild des Sensorsystems ist zu diesem Zweck eine Kompensationsimpedanz, die als Kapazität Ck ausgebildet ist, vorgesehen. Diese Kompensationsimpedanz wirkt zwischen der Elektrodeneinrichtung 10 und dem Signalausgang der Signalverarbeitungseinrichtung. Diese Kompensationsimpedanz Ck kann, wie in Figur 2 dargestellt, kapazitiv, aber auch resistiv oder induktiv sein. Durch das Vorsehen der Kompensationsimpedanz Ck lässt sich der Dynamikbereich des Sensorsystems vergrößern.

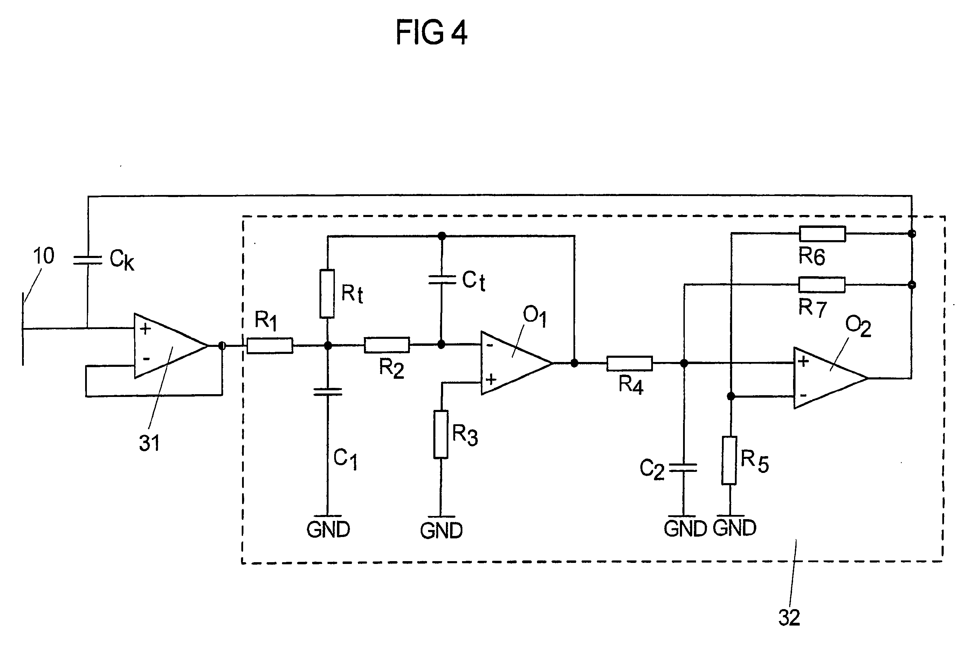

Eine weitere Ausführungsform einer Kompensationsschaltung unter Verwendung einer Kompensationsimpedanz Ck ist in der Figur 4 dargestellt. Die dargestellte Kompensationsschaltung weist eine Elektrodeneinrichtung 10, einen Impedanzwandler 31 und eine Schaltung 32, die zur Rückkopplung des Signals vom Ausgang der Signalverarbeitungseinrichtung 30 auf die Elektrodenvorrichtung 10 über die Kompensationsimpedanz Ck dient, auf. Die Schaltung 31 besteht aus zwei Stufen, von denen die erste Stufe, bestehend aus den Widerständen R1, R2, R3 und Rt, den Kapazitäten C1, Ct und dem Operationsverstärker O1, einen Tiefpass zweiter Ordnung und die zweite Stufe, bestehend aus dem Widerständen R4, R5, R6, R7, der Kapazität C2 und dem Operationsverstärker 02, eine Regelungsschaltung zur Rückkopplung des Signals auf die Elektrodeneinrichtung 10 darstellt. Ein von der Elektrodeneinrichtung 10 erfasstes Signal wird dann über den Impedanzwandler 31 zur Tiefpassanordnung geleitet, durch die Tiefpassanordnung gefiltert und über die Regelungsschaltung und die durch die Kapazität Ck gebildete Kompensationsimpedanz rückgekoppelt auf die Elektrodeneinrichtung 10.

Durch die Kompensationsimpedanz Ck wird bewirkt, dass ein Ladungsaustausch zwischen der Elektrodeneinrichtung 10 und dem Ausgang der Signalverarbeitungseinrichtung 30 erfolgen kann. Über die Kompensationsimpedanz Ck wird dabei ein Tiefpassgefiltertes Ausgangssignal mit entgegengesetztem Vorzeichen zur Elektrodeneinrichtung 10 rückgekoppelt, so dass genau die entgegengesetzte Ladungsmenge zur auf der Elektrodeneinrichtung 10 angesammelten Ladungsmenge auf die Elektrodeneinrichtung 10 eingekoppelt wird. Die Grenzfrequenz der Tiefpassanordnung ist dabei so klein gewählt, dass das Tiefpass-gefilterte Signal im Wesentlichen statischen Charakter hat, somit auch nur die niederfrequenten, im Wesentlichen statischen Anteile des Ausgangssignal auf die Elektrodeneinrichtung 10 zurückgekoppelt werden. Somit werden nur die niederfrequenten Ladungsanteile der Elektrodeneinrichtung 10 kompensiert, die (quasi)statischen Charakter aufweisen, also nur die im Wesentlichen statischen Ladungen, die sich auf der Elektrodeneinrichtung 10 angesammelt haben. Die Grenzfrequenz der Tiefpassanordnung kann dabei sinnvollerweise in der Größenordnung von 200 mHz liegen und somit deutlich unterhalb des Frequenzbereiches der zu erfassenden elektromagnetischen Signale aus einem Messobjekt Q.

Durch die in der Figur 2 und der Figur 4 dargestellte Kompensationsimpedanz Ck ist eine weitestgehend vollständige Unterdrückung der statischen Aufladung der Elektrodeneinrichtung 10 zu erreichen. Hierdurch ist eine Verbesserung des Dynamikbereichs der Elektrodeneinrichtung 10 sowie eine Vergrößerung des erreichbaren Signal-zu-Rausch-Verhältnisses des Sensorsystems möglich.

3. Verfahren zur Korrektur von Bewegungsartefakten mittels des Sensorsystems

Erfindungsgemäß wird ein Verfahren geschaffen, mittels dessen der Einfluss von Bewegungsartefakten auf ein gemessenes elektromagnetisches Signal aus einem Messobjekt Q, bewirkt durch eine relative Bewegung der kapazitiven Elektrodeneinrichtung 10 relativ zum Messobjekt Q, minimiert werden kann. Bei dem erfindungsgemäßen Verfahren ist dabei vorgesehen, dass ein Sensorsystem mit einer Elektrodeneinrichtung 10 oder eine Messvorrichtung mit einer Vielzahl von Sensorsystemen und Elektrodeneinrichtungen 10 an einem Messobjekt Q angebracht, ein elektrisches Wechselsignal über die Elektrodeneinrichtung 10 in das Messobjekt eingekoppelt, das eingekoppelte Wechselsignal ausgewertet und hierdurch die zeitliche Änderung der Elektrodenkapazität C der Elektrodeneinrichtung 10 des Sensorsystems ermittelt wird. Die Ermittlung der Elektrodenkapazität C erfolgt dabei für jede Elektrodeneinrichtung 10 eines jeden Sensorsystem separat, so dass die Bewegung einer jeden Elektrodeneinrichtung 10 gesondert kompensiert werden kann. Die Kompensation erfolgt, indem die zeitliche Änderung der Elektrodenkapazität bei der Auswertung der Messsignale eines jeden Sensorsystems berücksichtigt wird und dadurch die durch Bewegung bedingten Bewegungsartefakte aus dem Messsignal herausgerechnet werden.

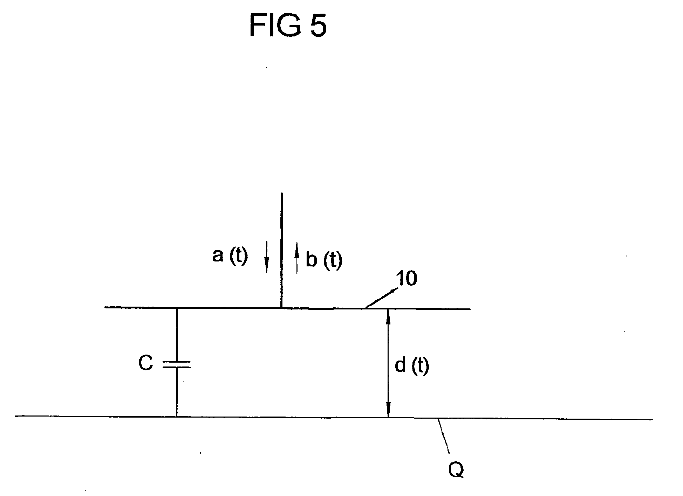

Eine Prinzipskizze der Anordnung einer Elektrodeneinrichtung 10 an einem Messobjekt Q ist in der Figur 5 dargestellt. Die Elektrodeneinrichtung 10 liegt dabei in einem Abstand d(t) vom Messobjekt Q, wobei der Abstand d(t) zeitlich variabel ist und somit auch die Kapazität C, die die Elektrodeneinrichtung 10 mit dem Messobjekt Q ausbildet, zeitlich veränderlich ist.

Um die Bewegungsartefakte zu bestimmen, wird ein zeitlich veränderliches Wechselsignal a(t) an die Elektrodeneinrichtung 10 angelegt und das Antwortsignal b(t) des Wechselsignals a(t) gemessen. Das Wechselsignal a(t) ist dabei ein Trägersignal bei einer bestimmten Frequenz, beispielsweise 300 Hz, während das Antwortsignal b(t) der Modulation des Wechselsignals a(t) durch die Bewegung der Elektrodeneinrichtung 10 relativ zum Messobjekt Q entspricht. Die Bewegung der Elektrodeneinrichtung 10 relativ zum Messobjekt Q ist dabei korreliert zur zeitlichen Änderung der Elektrodenkapazität C, so dass in dem durch das modulierte Wechselsignal a(t) gebildeten Antwortsignal b(t) die Information über die zeitliche Änderung der Elektrodenkapazität C enthalten ist.

In der in Figur 5 dargestellten Anordnung entspricht das Antwortsignal b(t) der Amplitudenmodulation des Wechselsignals a(t), bedingt durch die sich mit dem Abstand ändernde Elektrodenkapazität C. Es sind aber auch Anordnungen denkbar, bei denen das Wechselsignal a(t) durch die sich ändernde Elektrodenkapazität C frequenz- oder phasenmoduliert oder durch andere bekannte Modulationsverfahren moduliert wird, wobei zu diesem Zweck bekannte Schaltungen verwendet werden können, bei denen die Elektrodenkapazität C jeweils als modulierendes Bauteil fungiert.

Alternativ zur Einspeisung des Wechselsignals a(t) direkt über die Elektrodeneinrichtung 10 ist es auch möglich, das Wechselsignal a(t) über eine separate Referenzelektrode, die an anderer Stelle am Messobjekt Q angeordnet ist, in das Messobjekt Q einzukoppeln und über jede am Messobjekt Q angeordnete Elektrodeneinrichtung 10 ein Antwortsignal b(t) zu erfassen, das dann dem modulierten Anteil des Wechselsignal a(t) entspricht und für die jeweilige Elektrodeneinrichtung 10 die Information über die zeitliche Änderung der jeweiligen Elektrodenkapazität C enthält. Dieses ermöglicht eine Vereinfachung des Aufbaus des Sensorsystems, da in diesem Fall über jede Elektrodeneinrichtung 10 lediglich Signale empfangen werden müssen, die Einkopplung eines Wechselsignals a(t) aber über eine separate Referenzelektrode erfolgt. Ein Einspeisungspfad zur Einspeisung des Wechselsignals a(t) in jede Elektrodeneinrichtung 10 erübrigt sich in diesem Fall, so dass das Sensorsystem, wie in der Figur 2 dargestellt, lediglich einen Empfangspfad, also Mittel 30, 31, 32 zum Empfang eines Signals aufweisen muss. Eine solche Elektrodenanordnung kann beispielsweise so, wie in der Figur 3a bis c dargestellt, ausgebildet sein, wobei eine der Elektrodenelemente 100 dann als Referenzelektrode und die anderen Elektrodenelemente 100 als empfangende Elektroden dienen würden.

Die Referenzelektrode kann im Allgemeinen sowohl resistiv, induktiv oder kapazitiv an das Messobjekt Q angebracht sein, um ein Wechselsignal a(t) in das Messobjekt Q einzuspeisen. Es ist auch denkbar, mehrere Referenzelektroden zu verwenden, die Wechselsignale unterschiedlicher Frequenz einspeisen, wobei eine Elektrodeneinrichtung 10 jeweils ein Wechselsignal a(t) bei einer Frequenz empfängt, aus dem dann jeweils Rückschlüsse auf die Bewegung der jeweiligen Elektrodeneinrichtung 10 relativ zum Messobjekt Q gezogen werden können.

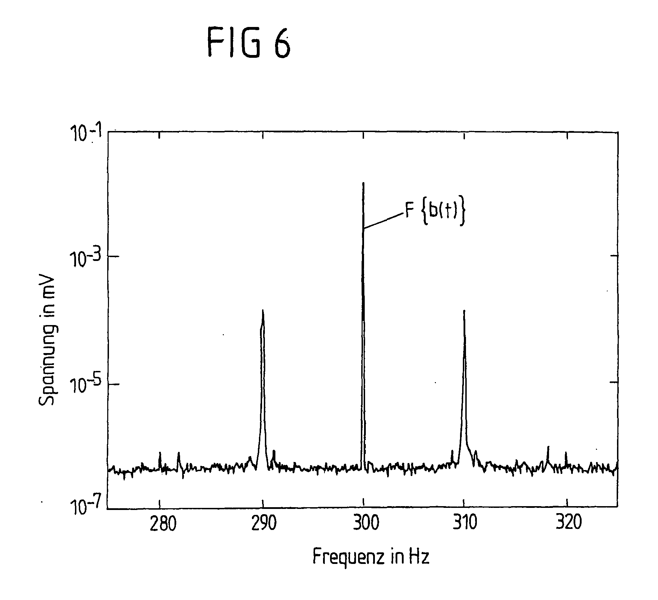

Ein Beispiel für ein durch eine Elektrodeneinrichtung 10 empfangenes Antwortsignal b(t) ist in der Figur 6 dargestellt. Hier gezeigt ist das Frequenzspektrum des Antwortsignals b(t), das der fouriertransformierten F{b(t)} des Antwortsignals b(t) entspricht. Im in der Figur 6 dargestellten Fall ist ein Wechselsignal a (t) über eine Referenzelektrode, die resistiv an dem Messobjekt Q angebracht ist, eingekoppelt worden, wobei die Elektrodeneinrichtung 10 eine Bewegung mit einer Frequenz von 10 Hz relativ zum Messobjekt Q ausführt. Dementsprechend weist das in der Figur 6 dargestellte Antwortsignal b(t) zwei Seitenbänder um die Frequenz des Wechselsignals a(t) von 300 Hz auf, nämlich bei 290 Hz und bei 310 Hz, die durch die Modulation des Wechselsignals a(t) durch die Bewegung der Elektrodeneinrichtung 10 relativ zum Messobjekt Q erzeugt werden.

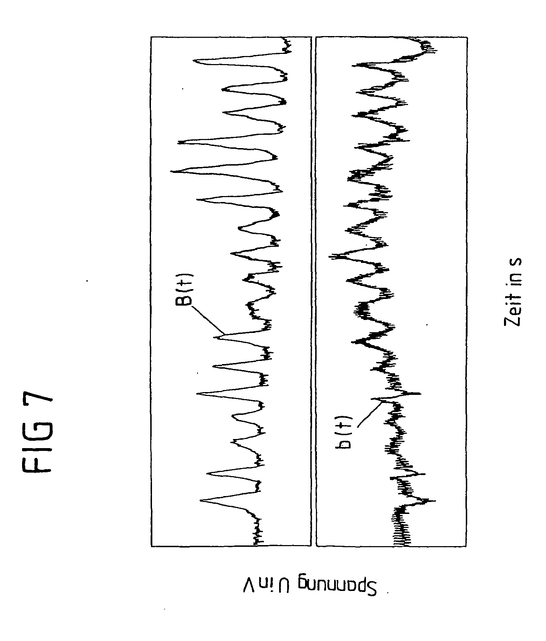

Figur 7 zeigt ein gemessenes Antwortsignal b(t) (unten in der Figur 7) und ein aus dem Antwortsignal b(t) errechnetes Bewegungssignal B(t) (oben in der Figur 7), das korreliert ist zur zeitlichen Änderung der Elektrodenkapazität C und somit die Information über die zeitliche Änderung der Elektrodeneinrichtung 10 relativ zum Messobjekt Q enthält. Das Bewegungssignal B(t) wird dabei aus dem Antwortsignal b(t) abgeleitet, indem das Antwortsignal b(t) Hochpassgefiltert wird und somit die Anteile des aus dem Messobjekt Q zu erfassenden elektromagnetischen Signals biologischen Ursprungs, die in einem Frequenzbereich unterhalb der Frequenz des Wechselsignals a(t), in diesem Fall 300 Hz, liegen, unterdrückt werden. Anschließend wird das Hochpass-gefilterte Antwortsignal b(t) demoduliert, somit der Anteil des ursprünglichen Wechselsignals a(t) aus dem Antwortsignal b(t) herausgerechnet und dadurch das Bewegungssignal B (t) bestimmt. Da das Bewegungssignal B(t) korreliert ist zur zeitlichen Änderung der Elektrodenkapazität C und somit die Information über die relative zeitliche Änderung der Elektrodenkapazität C in Abhängigkeit von der Bewegung der Elektrodeneinrichtung 10 relativ zum Messobjekt Q enthält, kann das Bewegungssignal B(t) weiterverarbeitet und mit bekannten Algorithmen der Signalverarbeitung zur Kompensation der Bewegungsartefakte im erfassten elektromagnetischen Messsignal biologischen Ursprungs verwendet werden. Die Kompensation der Bewegungsartefakte kann dabei entweder in einem der eigentlichen Messung nachgeordneten Post-Processing-Schritt durchgeführt werden oder auch bei entsprechend leistungsfähiger Signalverarbeitungseinrichtung 30 in Echtzeit während der Messung zur direkten Kompensation der Bewegungsartefakte ablaufen.

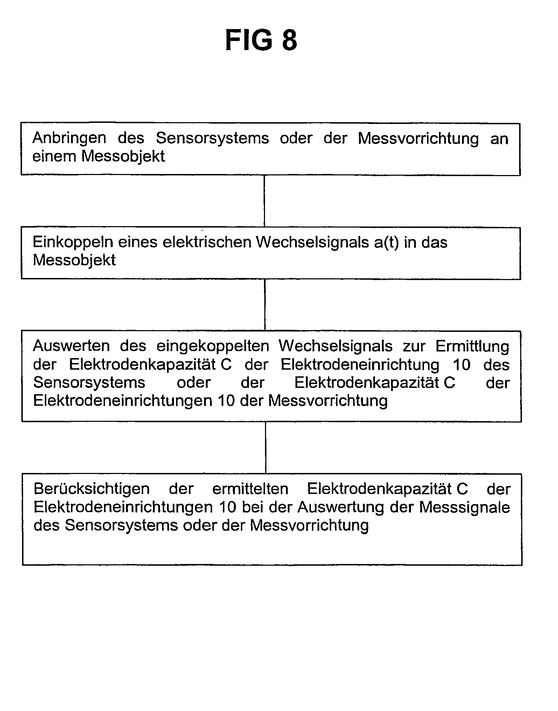

Figur 8 zeigt den grundlegenden Ablauf des Verfahrens zur Minimierung des Einflusses von Bewegungsartefakten unter Verwendung des erfindungsgemäßen Sensorsystems, bei dem die Änderung der Elektrodenkapazität C der Elektrodeneinrichtungen 10 bei der Auswertung der Messsignale des Sensorsystems oder der Messvorrichtung berücksichtigt wird.

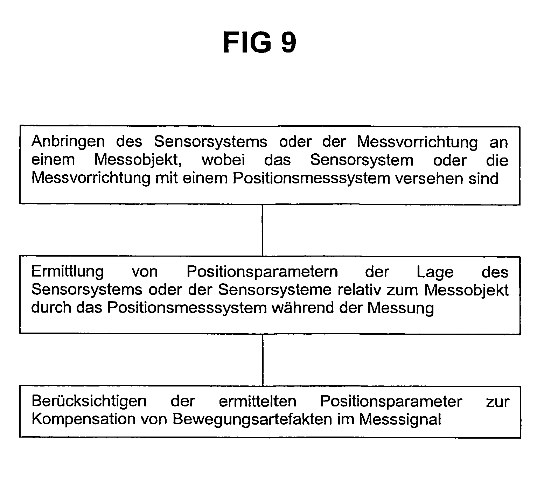

Der grundlegende Ablauf eines weiteren Verfahrens zur Minimierung des Einflusses von Bewegungsartefakten ist in der Figur 9 dargestellt. Bei diesem Verfahren wird zunächst das Sensorsystem oder die Messvorrichtung an einem Messobjekt angeordnet, wobei das Sensorsystem oder die Messvorrichtung mit einem Positionsmesssystem zur Bestimmung der Position und Lage des Sensorsystems oder Messvorrichtung versehen sind. Anschließend werden die Positionsparameter der Lage des Sensorsystems oder der Sensorsysteme relativ zum Messobjekt während der Messung ermittelt und die ermittelten Positionsparameter zur Kompensation von Bewegungsartefakten im Messsignal berücksichtigt.

**Patentansprüche**

1. Verfahren zur Minimierung des Einflusses von Bewegungsartefakten, unter Verwendung eines Sensorsystems mit einer kapazitiven Elektrodeneinrichtung (10) zur Messung elektromagnetischer Signale biologischen Ursprungs aufweisend

   - eine kapazitive Elektrodeneinrichtung (10),
   - ein die Elektrodeneinrichtung (10) zumindest teilweise umschließenden Elektroden-Abschirmelement (20) zur Abschirmung der Elektrodeneinrichtung (10) vor externen elektromagnetischen Störfeldern,
   - eine Signalverarbeitungseinrichtung (30) zur Verarbeitung elektromagnetischer Signale, die sich mittels der Elektrodeneinrichtung (10) detektieren lassen, und
   - zusätzliche Abschirmmittel (21), die zur Abschirmung externer elektromagnetischer Störfelder die Elektrodeneinrichtung (10) und das Elektroden-Abschirmelement (20) räumlich zumindest teilweise umschließen, wobei die zusätzlichen Abschirmmittel (21) mit unterschiedlichen Kompartimenten ausgebildet sind und in einem solchen Kompartiment die Signalverarbeitungseinrichtung (30) angeordnet ist,
   wobei das Verfahren folgende Schritte aufweist:
   - Anbringen des Sensorsystems an einem Messobjekt (Q),
   - Einkoppeln eines elektrischen Wechselsignals (a(t)) in das Messobjekt (Q),
   - Auswerten des eingekoppelten Wechselsignals zur Ermittlung der Elektrodenkapazität (C) der Elektrodeneinrichtung (10) des Sensorsystems und
   - Berücksichtigen der ermittelten Elektrodenkapazität (C) der Elektrodeneinrichtung (10) bei der Auswertung der Messsignale des Sensorsystems, indem die relative zeitliche Änderung der Elektrodenkapazität (C) der Elektrodeneinrichtung (10) des Sensorsystems berücksichtigt wird, aus der relativen zeitlichen Änderung der Elektrodenkapazität (C) ein Bewegungssignal (B(t)) der Elektrodeneinrichtung (10) relativ zum Messobjekt abgeleitet wird und mittels des ermittelten Bewegungssignals (B(t)) die die elektromagnetischen Signale biologischen Ursprungs überlagernden und störenden Bewegungsartefakte kompensiert werden.

2. Verfahren gemäß Anspruch 1, **dadurch gekennzeichnet, dass** das elektrische Wechselsignal zur Ermittlung der Elektrodenkapazität (C) der Elektrodeneinrichtung (10) des Sensorsystems über die Elektrodeneinrichtung (10) oder über eine externe mit dem Sensorsystem zusammenwirkende Referenzelektrode in das Messobjekt eingekoppelt wird.

3. Verfahren gemäß Anspruch 2, **dadurch gekennzeichnet, dass** die Referenzelektrode resistiv, induktiv oder kapazitiv mit dem Messobjekt (Q) gekoppelt ist.

4. Verfahren gemäß Anspruch 3, **dadurch gekennzeichnet, dass** eine oder mehrere Referenzelektroden verwendet werden.

5. Sensorsystem gemäß Anspruch 4, **dadurch gekennzeichnet, dass** mehrere Referenzelektroden zur Einkopplung von Wechselsignalen (a(t)) unterschiedlicher Frequenz verwendet werden, wobei mittels jedes einzelnen Wechselsignals (a(t)) die Bewegungsartefakte unterschiedlicher Elektrodenelemente (100) oder Elektrodeneinrichtungen (10) kompensierbar sind.

6. Verfahren gemäß einem der vorangehenden Ansprüche, **dadurch gekennzeichnet, dass** als elektrisches Wechselsignal ein netzfrequentes Störsignals benutzt wird.

7. Verfahren zur Minimierung des Einflusses von Bewegungsartefakten, unter Verwendung eines Sensorsystems mit einer kapazitiven Elektrodeneinrichtung (10) zur Messung elektromagnetischer Signale biologischen Ursprungs aufweisend

- eine kapazitive Elektrodeneinrichtung (10),
- ein die Elektrodeneinrichtung (10) zumindest teilweise umschließenden Elektroden-Abschirmelement (20) zur Abschirmung der Elektrodeneinrichtung (10) vor externen elektromagnetischen Störfeldern,
- eine Signalverarbeitungseinrichtung (30) zur Verarbeitung elektromagnetischer Signale, die sich mittels der Elektrodeneinrichtung (10) detektieren lassen, und
- zusätzliche Abschirmmittel (21), die zur Abschirmung externer elektromagnetischer Störfelder die Elektrodeneinrichtung (10) und das Elektroden-Abschirmelement (20) räumlich zumindest teilweise umschließen, wobei die zusätzlichen Abschirmmittel (21) mit unterschiedlichen Kompartimenten ausgebildet sind und in einem solchen Kompartiment die Signalverarbeitungseinrichtung (30) angeordnet ist,
wobei das Verfahren folgende Schritte aufweist:
- Anbringen des Sensorsystems an einem Messobjekt (Q), wobei das Sensorsystem mit einem optischen, akustischen oder piezoelektrischen Positionsmesssystem versehen sind,
- Ermitteln von Positionsparametern der Lage des Sensorsystems relativ zum Messobjekt (Q) durch das Positionsmesssystem während der Messung und
- Berücksichtigen der ermittelten Positionsparameter zur Kompensation von Bewegungsartefakten im Messsignal.

8. Verfahren gemäß einem der vorangehenden Ansprüche, **dadurch gekennzeichnet, dass** die Messsignale zum Entrauschen mittels digitaler Filter gefiltert werden, wobei die digitalen Filter an die momentane Signalcharakteristik adaptiert werden.

9. Verfahren gemäß Anspruch 8, **dadurch gekennzeichnet, dass** zusätzlich univariate oder multivariate Entrauschungsverfahren eingesetzt werden, die die Messsignale in Basissysteme zerlegen.

10. Verfahren nach einem der vorangehenden Ansprüche, **dadurch gekennzeichnet, dass** die Abschirmmittel (21) des Sensorsystems die Signalverarbeitungseinrichtung (30) zumindest teilweise derart umschließen, dass die Abschirmmittel (21) auch als elektromagnetische Abschirmung zwischen Signalverarbeitungseinrichtung (30) und Elektrodeneinrichtung (10) wirken.

11. Verfahren nach einem der vorangehenden Ansprüche, **dadurch gekennzeichnet, dass** die Signalverarbeitungseinrichtung (30) des Sensorsystems eine als Impedanzwandler ausgebildete Eingangsstufe (31) aufweist.

12. Verfahren nach einem der vorangehenden Ansprüche, **dadurch gekennzeichnet, dass** die Signalverarbeitungseinrichtung (30) des Sensorsystems einen Differenzverstärker aufweist, wobei als Differenzsignal das Signal einer externen Referenzelektrode verarbeitbar ist.

13. Verfahren nach einem der vorangehenden Ansprüche, **dadurch gekennzeichnet, dass** die Signalverarbeitungseinrichtung (30) des Sensorsystems einen Analog-Digital-Wandler und einen digitalen Signalprozessor umfasst.

14. Verfahren nach einem der vorangehenden Ansprüche, **dadurch gekennzeichnet, dass** über eine kapazitiv, resistiv oder induktiv realisierte Kompensationsimpedanz (Ck) eine Rückkopplung verarbeiteter Signale auf die Elektrodeneinrichtung (10) vorgesehen ist.

15. Verfahren nach einem der vorangehenden Ansprüche, **dadurch gekennzeichnet, dass** die Eingangsimpedanz der Signalverarbeitungseinrichtung (30) derart gewählt ist, dass sich zusammen mit der Elektrodenkapazität (C) des Sensorsystems ein Hochpass mit einer an ein zu messendes elektromagnetisches Signal biologischen Ursprungs angepassten Grenzfrequenz ausbildet.

16. Verfahren nach einem der vorangehenden Ansprüche, **dadurch gekennzeichnet, dass** die Elektrodeneinrichtung (10) des Sensorsystems eine Mehrzahl von Elektrodenelementen (100) zum Detektieren elektromagnetischer Signale biologischen Ursprungs aufweist, wobei die Signalverarbeitungseinrichtung als entsprechende Mehrzahl parallel geschalteter Teil-Signalverarbeitungseinrichtungen ausgebildet ist.

17. Verfahren nach einem der vorangehenden Ansprüche, **dadurch gekennzeichnet, dass** das Sensorsystem Mittel zum leitungsgebundenen und/oder Mittel zum leitungslosen Übertragen der Messsignale vom Sensorsystem zu einer davon beabstandeten Empfangseinrichtung aufweist.

18. Verfahren nach einem der vorangehenden Ansprüche, **dadurch gekennzeichnet, dass** eine Messvorrichtung mit einer Vielzahl von Sensorsystemen verwendet wird, wobei die Sensorsysteme in einer helm- oder kappenartigen Trägereinrichtung angeordnet sind, die zumindest abschnittsweise über den Kopf einer Testperson zu stülpen ist.

19. Verfahren nach einem der vorangehenden Ansprüche, **dadurch gekennzeichnet, dass** eine Messvorrichtung mit einer Vielzahl von Sensorsystemen verwendet wird, wobei die Sensorsysteme an einer flexiblen flächig ausgebildeten Trägereinrichtung angeordnet sind, die am Körper einer Testperson zu befestigen ist.

**Claims**

1. A method for minimizing the influence of movement artefacts, by using a sensor system with a capacitive electrode device (10) for measuring electromagnetic signals having a biological origin, comprising

   - a capacitive electrode device (10),
   - an electrode shielding element (20), at least partially surrounding the electrode device (10), for shielding the electrode device (10) against external electromagnetic interference fields,
   - a signal processing device (30) for processing electromagnetic signals that can be detected by means of the electrode device (10), and
   - additional shielding means (21) which for shielding out external electromagnetic interference fields at least partially surround the electrode device (10) and the electrode shielding element (20) in three dimensions, the additional shielding means (21) being formed with different compartments, and the signal processing device (30) being arranged in one such compartment, wherein the method comprises the following steps:
   - fitting the sensor system on a measurement object (Q),
   - coupling an electrical alternating signal (a(t)) into the measurement object (Q),
   - evaluating the alternating signal coupled in, in order to determine the electrode capacitance (C) of the electrode device (10) of the sensor system, and
   - taking account of the determined electrode capacitance (C) of the electrode device (10) when evaluating the measurement signals of the sensor system, in that the relative temporal change of the electrode capacitance (C) of the electrode device (10) of the sensor system is taken into account, a movement signal (B(t)) of the electrode device (10) relative to the measurement object is derived from the relative temporal change of the electrode capacitance (C), and the interfering movement artefacts superposed on the electromagnetic signals having a biological origin are compensated by means of the determined movement signal (B(t)).

2. The method according to claim 1, **characterized in that** the electrical alternating signal for determining the electrode capacitance (C) of the electrode device (10) of the sensor system is coupled into the measurement object via the electrode device (10) or via an external reference electrode cooperating with the sensor system.

3. The method according to claim 2, **characterized in that** the reference electrode is coupled with the measurement object (Q) in a resistive, inductive or capacitive fashion.

4. The method according to claim 3, **characterized in that** one or more reference electrodes are used.

5. The sensor system according to claim 4, **characterized in that** a plurality of reference electrodes are used for coupling in alternating signals (a(t)) of different frequencies, wherein by means of each individual alternating signal (a(t)) the movement artefacts of different electrode elements (100) or electrode devices (10) can be compensated.

6. The method according to any of the preceding claims, **characterized in that** a line frequency interference signal is used as electrical alternating signal.

7. A method for minimizing the influence of movement artefacts, by using a sensor system with a capacitive electrode device (10) for measuring electromagnetic signals having a biological origin, comprising

- a capacitive electrode device (10),
- an electrode shielding element (20), at least partially surrounding the electrode device (10), for shielding the electrode device (10) against external electromagnetic interference fields,
- a signal processing device (30) for processing electromagnetic signals that can be detected by means of the electrode device (10), and
- additional shielding means (21) which for shielding out external electromagnetic interference fields at least partially surround the electrode device (10) and the electrode shielding element (20) in three dimensions, the additional shielding means (21) being formed with different compartments, and the signal processing device (30) being arranged in one such compartment, wherein the method comprises the following steps:
- fitting the sensor system on a measurement object (Q), the sensor system being provided with an optical, acoustic or piezoelectric position measuring system,
- determining position parameters of the position of the sensor system relative to the measurement object (Q) by means of the position measuring system during the measurement, and
- taking account of the determined position parameters, in order to compensate movement artefacts in the measurement signal.

8. The method according to any of the preceding claims, **characterized in that** the measurement signals are filtered for denoising purposes by means of digital filters, the digital filters being adapted to the instantaneous signal characteristic.

9. The method according to claim 8, **characterized in that** use is made in addition of univariate or multivariate denoising methods that decompose the measurement signals into base systems.

10. The method according to any of the preceding claims, **characterized in that** the shielding means (21) of the sensor system at least partially surround the signal processing device (30) in such a way that the shielding means (21) also act as an electromagnetic shield between signal processing device (30) and electrode device (10).

11. The method according to any of the preceding claims, **characterized in that** the signal processing device (30) of the sensor system includes an input stage (31) designed as impedance converter.

12. The method according to any of the preceding claims, **characterized in that** the signal processing device (30) of the sensor system includes a difference amplifier, wherein the signal of an external reference electrode can be processed as difference signal.

13. The method according to any of the preceding claims, **characterized in that** the signal processing device (30) of the sensor system comprises an analog-digital converter and a digital signal processor.

14. The method according to any of the preceding claims, **characterized in that** a feedback of processed signals to the electrode device (10) is provided via a compensation impedance (Ck) implemented in a capacitive, resistive or inductive fashion.

15. The method according to any of the preceding claims, **characterized in that** the input impedance of the signal processing device (30) is chosen such that together with the electrode capacitance (C) of the sensor system a high-pass filter with a cutoff frequency adapted to an electromagnetic signal, having a biological origin, to be measured is formed.

16. The method according to any of the preceding claims, **characterized in that** the electrode device (10) of the sensor system includes a plurality of electrode elements (100) for detecting electromagnetic signals having a biological origin, wherein the signal processing device is designed as a corresponding plurality of partial signal processing devices connected in parallel.

17. The method according to any of the preceding claims, **characterized in that** the sensor system includes means for the line-bound transmission and/or means for the lineless transmission of the measurement signals from the sensor system to a receiving means spaced apart therefrom.

**18.** The method according to any of the preceding claims, **characterized in that** a measurement device with a plurality of sensor systems is used, wherein the sensor systems are arranged in a helmet-like or cap-like carrier device that can at least partially be slipped over the head of a test subject.

**19.** The method according to any of the preceding claims, **characterized in that** a measuring device with a plurality of sensor systems is used, wherein the sensor systems are arranged on a flexible carrier device of two-dimensional design that can be fastened on the body of a test subject.

**Revendications**

**1.** Procédé pour réduire l'influence d'artefacts de mouvement, en employant un système de détection ayant un dispositif d'électrode capacitive (10) servant à mesurer des signaux électromagnétiques d'origine biologique, comprenant

   - un dispositif d'électrode capacitive (10),
   - un élément de protection d'électrode (20) enfermant au moins partiellement le dispositif d'électrode (10), afin de protéger ledit dispositif d'électrode (10) contre des champs électromagnétiques externes parasites,
   - un dispositif de traitement de signaux (30) destiné à traiter des signaux électromagnétiques qui peuvent être détectés au moyen du dispositif d'électrode (10), et
   - des moyens de protection supplémentaires (21) qui enferment spatialement au moins en partie le dispositif d'électrode (10) et l'élément de protection d'électrode (20) à des fins de protection contre des champs électromagnétiques externes parasites, les moyens de protection supplémentaires (21) étant formés avec différents compartiments et le dispositif de traitement de signaux (30) étant agencé dans un tel compartiment,
   le procédé comportant les étapes suivantes consistant à :
   - appliquer le système de détection contre un objet à mesurer (Q),
   - envoyer un signal électrique alternatif (a(t)) dans l'objet à mesurer (Q),
   - évaluer le signal alternatif envoyé afin de déterminer la capacité de l'électrode (C) du dispositif d'électrode (10) du système de détection et
   - tenir compte de la capacité déterminée de l'électrode (C) du dispositif d'électrode (10) lors de l'évaluation des signaux de mesure du système de détection par le fait que l'on tienne compte de la variation temporelle relative de la capacité de l'électrode (C) du dispositif d'électrode (10) du système de détection, par le fait que l'on déduise un signal de mouvement (B(t)) du dispositif d'électrode (10) relativement à l'objet à mesurer à partir de la variation temporelle relative de la capacité de l'électrode (C), et par le fait que l'on compense les artefacts de mouvement, parasites et superposés aux signaux électromagnétiques d'origine biologique, au moyen du signal de mouvement déterminé (B(t)).

**2.** Procédé selon la revendication 1, **caractérisé en ce que** le signal électrique alternatif servant à déterminer la capacité de l'électrode (C) du dispositif d'électrode (10) du système de détection est envoyé dans l'objet à mesurer par le biais du dispositif d'électrode (10) ou par le biais d'une électrode de référence externe coopérant avec le système de détection.

**3.** Procédé selon la revendication 2, **caractérisé en ce que** l'électrode de référence est couplée de manière résistive, inductive ou capacitive avec l'objet à mesurer (Q).

**4.** Procédé selon la revendication 3, **caractérisé en ce que** l'on emploie une ou plusieurs électrodes de référence.

**5.** Système de détection selon la revendication 4, **caractérisé en ce que** plusieurs électrodes de référence sont employées pour envoyer des signaux alternatifs (a(t)) de fréquences différentes, les artefacts de mouvement de différents éléments d'électrode (100) ou dispositifs d'électrode (10) pouvant être compensés au moyen de chaque signal alternatif particulier (a(t)).

**6.** Procédé selon l'une quelconque des revendications précédentes, **caractérisé en ce qu'**un signal parasite à la fréquence du réseau est employé en tant que signal électrique alternatif.

**7.** Procédé pour réduire l'influence d'artefacts de mouvement, en employant un système de détection ayant un dispositif d'électrode capacitif (10) pour mesurer des signaux électromagnétiques d'origine biologique comportant :

   - un dispositif d'électrode capacitive (10),

- un élément de protection d'électrode (20) enfermant au moins partiellement le dispositif d'électrode (10) afin de protéger ledit dispositif d'électrode (10) contre des champs électromagnétiques externes parasites,
- un dispositif de traitement de signaux (30) destiné à traiter des signaux électromagnétiques qui peuvent être détectés au moyen du dispositif d'électrode (10), et
- des moyens de protection supplémentaires (21) qui enferment spatialement au moins en partie le dispositif d'électrode (10) et l'élément de protection d'électrode (20) à des fins de protection contre des champs électromagnétiques externes parasites, les moyens de protection supplémentaires (21) étant formés avec différents compartiments et le dispositif de traitement de signaux (30) étant agencé dans un tel compartiment, le procédé comportant les étapes suivantes consistant à :
- appliquer le système de détection contre un objet à mesurer (Q), le système de détection étant équipé d'un système de mesure de position optique, acoustique ou piézoélectrique,
- déterminer des paramètres de position pour la position du système de détection relativement à l'objet à mesurer (Q) par le biais du système de mesure de position durant la mesure et
- tenir compte des paramètres de position déterminés pour compenser des artefacts de mouvement dans le signal de mesure.

**8.** Procédé selon l'une quelconque des revendications précédentes, **caractérisé en ce que** les signaux de mesure sont filtrés au moyen de filtres numériques pour supprimer le bruit, les filtres numériques étant adaptés à la caractéristique de signal instantanée.

**9.** Procédé selon la revendication 8, **caractérisé en ce que** l'on met en oeuvre des procédés de suppression de bruit univariés ou multivariés qui fractionnent les signaux de mesure en des systèmes de base.

**10.** Procédé selon l'une quelconque des revendications précédentes, **caractérisé en ce que** les moyens de protection (21) du système de détection enferment au moins partiellement le dispositif de traitement de signaux (30) de manière telle que les moyens de protection (21) aient également un effet de protection électromagnétique entre le dispositif de traitement de signaux (30) et le dispositif d'électrode (10).

**11.** Procédé selon l'une quelconque des revendications précédentes, **caractérisé en ce que** le dispositif de traitement de signaux (30) du système de détection comporte un étage d'entrée (31) réalisé sous la forme d'un convertisseur d'impédance.

**12.** Procédé selon l'une quelconque des revendications précédentes, **caractérisé en ce que** le dispositif de traitement de signaux (30) du système de détection comporte un amplificateur différentiel, le signal d'une électrode de référence externe pouvant être traité en tant que signal différentiel.

**13.** Procédé selon l'une quelconque des revendications précédentes, **caractérisé en ce que** le dispositif de traitement de signaux (30) du système de détection comporte un convertisseur analogique-numérique et un processeur de signaux numériques.

**14.** Procédé selon l'une quelconque des revendications précédentes, **caractérisé en ce que**, sur le dispositif d'électrode (10), il est prévu un rétrocouplage des signaux traités par le biais d'une impédance de compensation (Ck) réalisée de manière capacitive, résistive ou inductive.

**15.** Procédé selon l'une quelconque des revendications précédentes, **caractérisé en ce que** l'impédance d'entrée du dispositif de traitement de signaux (30) est choisie de manière à former conjointement avec la capacité de l'électrode (C) du système de détection un filtre passe-haut ayant une fréquence de coupure adaptée au signal électromagnétique d'origine biologique à mesurer.

**16.** Procédé selon l'une quelconque des revendications précédentes, **caractérisé en ce que** le dispositif d'électrode (10) du système de détection comporte une pluralité d'éléments d'électrode (100) servant à détecter des signaux électromagnétiques d'origine biologique, le dispositif de traitement de signaux étant réalisé sous la forme d'une pluralité correspondante de dispositifs partiels de traitement de signaux agencés en parallèle.

**17.** Procédé selon l'une quelconque des revendications précédentes, **caractérisé en ce que** le système de détection comporte des moyens pour transmettre par fils et/ou des moyens pour transmettre sans fil les signaux de mesure du système de détection à un dispositif de réception distant dudit système de détection.

**18.** Procédé selon l'une quelconque des revendications précédentes, **caractérisé en ce que** l'on emploie un dispositif de mesure ayant une multitude de systèmes de détection, lesdits systèmes de détection étant agencés dans un dispositif de support en forme de casque ou de casquette qui doit être placé au moins par sections sur la tête d'une personne à examiner.

**19.** Procédé selon l'une quelconque des revendications précédentes, **caractérisé en ce que** l'on emploie un dispositif de mesure ayant une multitude de systèmes de détection, lesdits systèmes de détection étant agencés sur un dispositif de support flexible de forme plane qui doit être fixé sur le corps d'une personne à examiner.

## FIG 1A

## FIG 1B

FIG 2

EP 1 827 218 B1

## FIG 3A

## FIG 3B

## FIG 3C

EP 1 827 218 B1

FIG 4

# FIG 5

# FIG 6

## FIG 7

B(t)

b(t)

Spannung U in V

Zeit in s

# FIG 8

Anbringen des Sensorsystems oder der Messvorrichtung an einem Messobjekt

Einkoppeln eines elektrischen Wechselsignals a(t) in das Messobjekt

Auswerten des eingekoppelten Wechselsignals zur Ermittlung der Elektrodenkapazität C der Elektrodeneinrichtung 10 des Sensorsystems oder der Elektrodenkapazität C der Elektrodeneinrichtungen 10 der Messvorrichtung

Berücksichtigen der ermittelten Elektrodenkapazität C der Elektrodeneinrichtungen 10 bei der Auswertung der Messsignale des Sensorsystems oder der Messvorrichtung

# FIG 9

| Anbringen des Sensorsystems oder der Messvorrichtung an einem Messobjekt, wobei das Sensorsystem oder die Messvorrichtung mit einem Positionsmesssystem versehen sind |
|---|

| Ermittlung von Positionsparametern der Lage des Sensorsystems oder der Sensorsysteme relativ zum Messobjekt durch das Positionsmesssystem während der Messung |
|---|

| Berücksichtigen der ermittelten Positionsparameter zur Kompensation von Bewegungsartefakten im Messsignal |
|---|

**EP 1 827 218 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20030036691 A1
- WO 03048789 A2
- US 2004254435 A1
- US 2004073104 A1
- US 6445940 B1

### In der Beschreibung aufgeführte Nicht-Patentliteratur

- **C. J. Harland et al.** Remote detection of human electroencephalograms using ultrahigh input impedance electric potential sensors. *Applied Physics Letters,* vol. 81 (17), 21.10.2402